# EUROPEAN PATENT APPLICATION

(11) **EP 4 772 187 A1**
(43) Date of publication of application: **08.07.2026**
(21) Application number: 24858733.9
(22) Date of filing: 30.08.2024
(51) Int. Cl.: A61K 36/605, A61P 5/16, A61P 43/00, A61P 29/00

(54) **USE OF MULBERRY EXTRACT IN PREPARATION OF DRUG FOR ALLEVIATING AND/OR TREATING THYROID DISEASES**

(30) Priority: 01.09.2023 CN 202311122393; 06.03.2024 CN 202410254236
(71) Applicant: Beijing Wehand-Bio Pharmaceutical Co., Ltd, Beijing 102600 (CN); Guangxi Wehand-Bio Pharmaceutical Co., Ltd, Hechi, Guangxi 546300 (CN)
(72) Inventor: LIU, Yuling, Beijing 102600 (CN); LIU, Yuanyuan, Beijing 102600 (CN); WANG, Xingang, Beijing 102600 (CN); LIU, Dongdong, Beijing 102600 (CN); LI, Huijuan, Beijing 102600 (CN); LIU, Zhihua, Beijing 102600 (CN); CHEN, Yanmin, Beijing 102600 (CN); WANG, Tingting, Beijing 102600 (CN); LI, Na, Beijing 102600 (CN); ZOU, Yuanyuan, Beijing 102600 (CN); CAO, Haiyan, Beijing 102600 (CN); GAO, Lili, Beijing 102600 (CN)
(74) Representative: Winter, Brandl - Partnerschaft mbB
(86) International application number: PCT/CN2024/115874
(87) International publication number: WO 2025/045202

(57) **Abstract**

The present disclosure discloses the use of mulberry extract in preparing a medicament for improving and/or treating thyroid disease. This use refers to the use of mulberry extract in preparing a product for treating and/or improving thyroid disease. It has been confirmed by experiments in the present disclosure that mulberry extract can regulate the secretion levels of TSH, FT3, and FT4 in SD rats with hyperthyroidism or hypothyroidism, regulate the levels of thyroid antibodies in patients with Hashimoto's thyroiditis, and has a good improving effect on thyroid tissue lesions. As a component derived from natural plants, the mulberry extract of the present disclosure has unique advantages of small toxic and side effects and mild and long-lasting effects. Therefore, the mulberry extract with greatly improved medication safety has great advantages in the treatment of thyroid diseases

## Description

### CROSS-REFERENCE TO RELATED APPLICATIONS

This application claims the priority of the Chinese patent application filed on September 1, 2023 (Application No. 202311122393.9) and the Chinese patent application filed on March 6, 2024 (Application No. 202410254236.1). The entire contents of the aforementioned patent applications are hereby incorporated by reference into this application.

### TECHNICAL FIELD

The present disclosure belongs to the medical field and specifically relates to use of mulberry extract in preparing a medicament for improving and/or treating thyroid disease.

### BACKGROUND

The thyroid is a very important endocrine gland in the human body. It produces, secretes, and stores thyroid hormones (TH) through thyroid follicular epithelial cells, which act on the body to regulate metabolism and affect almost all oxygen-consuming tissues. TH includes thyroxine (T4) and triiodothyronine (T3), most of which are bound to plasma proteins, and only the free forms are biologically active, namely free triiodothyronine (FT3) and free thyroxine (FT4). The secretion of TH is regulated by the hypothalamus-pituitary-thyroid axis. The hypothalamus produces thyrotropin-releasing hormone, which acts on the pituitary gland. The pituitary gland then releases thyroid-stimulating hormone (TSH), which acts on the thyroid gland, stimulating it to release TH into the body. In turn, TH negatively feeds back to the pituitary gland to inhibit the secretion of TSH. Therefore, TSH, FT3, and FT4 are commonly used in clinical practice to evaluate thyroid function.

Thyroid diseases are mainly classified into hyperthyroidism, hypothyroidism, thyroid nodules, and thyroiditis. In hyperthyroidism, an excessive amount of thyroid hormone is secreted, while in hypothyroidism, a small amount of thyroid hormone is secreted. Thyroid nodules are caused by the abnormal proliferation of thyroid cells. Studies have shown that abnormal thyroid function has a serious impact on the body's growth, metabolism, and reproduction.

Hashimoto's thyroiditis (HT) is a common clinical autoimmune thyroid disease, characterized mainly by the presence of thyroid-specific autoantibodies and lymphocytic infiltration in the thyroid gland, ultimately leading to the destruction of thyroid tissue structure and hypothyroidism. In the early stage of the disease, antibodies secreted by the thyroid gland itself attack it, causing a large amount of already synthesized thyroid hormone to leak into the blood, resulting in a "hyperthyroidism" state. In the later stage, as the previously synthesized thyroid hormone is depleted and the thyroid gland is damaged, the synthesis of thyroxine becomes limited, leading to a "hypothyroidism" state. In HT patients, thyroid function often changes according to the development pattern of "normal → hyperthyroidism → hypothyroidism." Clinical manifestations include general weakness, unilateral or bilateral diffuse goiter, especially thickening of the isthmus, lymphocytic infiltration, fibrosis, interstitial atrophy, and oxyphilic changes of acinar cells in the thyroid gland. Autoantibodies such as anti-thyroglobulin antibody (TG-Ab) and anti-thyroid peroxidase antibody (TPO-Ab) appear in the patient's serum. In a few patients, thyroid-stimulating hormone (TSH) is elevated, and free thyroxine (FT4) is decreased.

Currently, clinical treatments for thyroid-related diseases mainly include thyroid hormone replacement therapy, antithyroid drugs, and surgery. However, these treatments require long-term medication, have strong dependency, and cause significant adverse reactions. Although drug treatments such as synthetic hormones have been used to treat hyperthyroidism and hypothyroidism, these drugs all have side effects and limitations in clinical application.

### SUMMARY

The present disclosure provides the use of mulberry extract in the preparation of a medicament for improving and/or treating thyroid disease. The present disclosure confirms through experiments that mulberry extract can regulate the secretion levels of TSH, FT3, and FT4 in Sprague-Dawley (SD) rats with hyperthyroidism or hypothyroidism, regulate the levels of thyroid antibodies in patients with Hashimoto's thyroiditis, and has a significant improving effect on thyroid tissue lesions. As a component derived from natural plants, the mulberry extract of the present disclosure possesses the unique advantages of minimal toxic and side effects, as well as mild and long-lasting actions. Therefore, the mulberry extract, with its greatly improved medication safety, has significant advantages in the treatment of thyroid diseases.

### Technical Solution

The objective of the present disclosure is to provide a new pharmaceutical use of mulberry extract or its main active ingredient.

The new use of mulberry extract or its main active ingredient provided by the present disclosure is any one of the following (a1) to (a4):
(a1) use of mulberry extract in preparing a product for preventing and/or treating thyroid disease;
(a2) use of mulberry extract in preparing a product for improving thyroid disease;
(a3) use of mulberry extract in preventing and/or treating thyroid disease;
(a4) use of mulberry extract in improving thyroid disease.

The thyroid disease comprises at least one of the following: hypothyroidism, hyperthyroidism, thyroid nodule, and thyroiditis.

Preferably, the thyroiditis comprises Hashimoto's thyroiditis.

The treating and/or improving of thyroid disease is reflected in at least one of the following aspects:
1) regulating the secretion level of serum thyroid-stimulating hormone (TSH) in a patient with thyroid disease;
2) regulating the secretion level of free triiodothyronine (free triiodothyronine, FT3) in a patient with thyroid disease;
3) regulating the secretion level of free thyroxine (FT4) in a patient with thyroid disease;
4) improving thyroid tissue lesions in a patient with thyroid disease;
5) improving the level of thyroid antibodies in a patient with thyroid disease;
6) reducing the level of reactive oxygen species (ROS) in a patient with thyroid disease;
7) improving the level of inflammatory factors in a patient with thyroid disease.

Further, the thyroid antibody is TPO-Ab antibody and/or TG-Ab antibody.

Further, the inflammatory factor is at least one of the following: TNF-α, IL-1β, and IL-6.

In the present disclosure, the thyroid disease is preferably hypothyroidism and/or hyperthyroidism.

Further, the hypothyroidism may be hyperandrogenism-related endocrine disease or endocrine disease related to glucose and lipid metabolism disorder accompanied by hypothyroidism. Alternatively, the hypothyroidism may be caused by hyperandrogenism or its related endocrine diseases, alternatively the hypothyroidism may be caused by glucose and lipid metabolism disorder. The hyperandrogenism-related endocrine disease may be hyperandrogenemia or PCOS.

The hypothyroidism is reflected in a disorder of the serum thyroid-stimulating hormone (TSH) level, with or without a decrease in the level of free triiodothyronine (FT3) and/or free thyroxine (FT4). Alternatively, the hypothyroidism is reflected in a normal level of serum thyroid-stimulating hormone (TSH) and a decrease in the level of free thyroxine (FT4), with or without a decrease in the level of free triiodothyronine (FT3). The disorder of the TSH level comprises an increase or decrease in the TSH level.

Furthermore, the hypothyroidism is reflected in an increase in the level of serum thyroid-stimulating hormone (TSH). Alternatively, the hypothyroidism is reflected in an increase in the level of serum thyroid-stimulating hormone (TSH) and a decrease in the level of free thyroxine (FT4). Alternatively, the hypothyroidism is reflected in an increase in the level of serum thyroid-stimulating hormone (TSH) and decreases in the levels of free triiodothyronine (FT3) and free thyroxine (FT4).

Alternatively, the hypothyroidism is reflected in a decrease in the level of serum thyroid-stimulating hormone (TSH) and decreases in the levels of free triiodothyronine (FT3) and free thyroxine (FT4).

Alternatively, the hypothyroidism is reflected in a normal level of serum thyroid-stimulating hormone (TSH) and a decrease in the level of free thyroxine (FT4). Alternatively, the hypothyroidism is reflected in a normal level of serum thyroid-stimulating hormone (TSH) and decreases in the levels of free triiodothyronine (FT3) and free thyroxine (FT4).

Furthermore, the preventing and/or treating and/or improving of hypothyroidism is reflected in regulating the TSH level, and/or increasing the level of FT3, and/or increasing the level of FT4. The "regulating" is increasing, decreasing, or maintaining a normal level.

Specifically, the preventing and/or treating and/or improving of hypothyroidism may be reflected in decreasing the TSH level and increasing the levels of FT3 and FT4.

Further, the hyperthyroidism may be hyperandrogenism-related endocrine disease or endocrine disease related to glucose and lipid metabolism accompanied by hyperthyroidism. Alternatively, the hyperthyroidism may be caused by hyperandrogenism or its related endocrine diseases, or the hyperthyroidism may be caused by glucose and lipid metabolism disorder. The hyperandrogenism-related endocrine disease may be hyperandrogenemia or PCOS.

The hyperthyroidism is reflected in a disorder of the serum thyroid-stimulating hormone (TSH) level, with or without an increase in the level of free triiodothyronine (FT3) and/or free thyroxine (FT4). Alternatively, the hyperthyroidism is reflected in a normal level of serum thyroid-stimulating hormone (TSH) and increases in the levels of free triiodothyronine (FT3) and free thyroxine (FT4). The disorder of the TSH level comprises an increase or decrease in the TSH level.

Furthermore, the hyperthyroidism is reflected in a decrease in the level of serum thyroid-stimulating hormone (TSH). Alternatively, the hyperthyroidism is reflected in a decrease in the level of serum thyroid-stimulating hormone (TSH) and an increase in the level of free thyroxine (FT4). Alternatively, the hyperthyroidism is reflected in a decrease in the level of serum thyroid-stimulating hormone (TSH) and increases in the levels of free triiodothyronine (FT3) and free thyroxine (FT4). Alternatively, the hyperthyroidism is reflected in a decrease in the level of serum thyroid-stimulating hormone (TSH) and an increase in the level of free triiodothyronine (FT3).

Alternatively, the hyperthyroidism is reflected in an increase in the level of serum thyroid-stimulating hormone (TSH) and increases in the levels of free triiodothyronine (FT3) and free thyroxine (FT4).

Alternatively, the hyperthyroidism is reflected in a normal level of serum thyroid-stimulating hormone (TSH) and increases in the levels of free triiodothyronine (FT3) and free thyroxine (FT4).

Furthermore, the preventing and/or treating and/or improving of hyperthyroidism is reflected in the following aspects: regulating the TSH level, and/or decreasing the level of FT3, and/or decreasing the level of FT4. The "regulating" is increasing, decreasing, or maintaining a normal level.

Specifically, the preventing and/or treating and/or improving of hyperthyroidism may be reflected in the following aspects: increasing the TSH level and decreasing the levels of FT3 and FT4.

Further, the Hashimoto's thyroiditis is reflected in at least one of the following aspects: a significant increase in the levels of serum TPO-Ab and TG-Ab antibodies.

Furthermore, the preventing and/or treating and/or improving of Hashimoto's thyroiditis is reflected in at least one of the following aspects: reducing the level of serum TPO-Ab antibodies and reducing the level of serum TG-Ab antibodies.

The present disclosure also protects the use of mulberry extract or its main active ingredient, which is at least one of the following (b1) to (b7):
(b1) preparing a product for regulating the secretion level of serum thyroid-stimulating hormone (TSH) in a patient with thyroid disease;
(b2) preparing a product for regulating the secretion level of free triiodothyronine (FT3) in a patient with thyroid disease;
(b3) preparing a product for regulating the secretion level of free thyroxine (FT4) in a patient with thyroid disease;
(b4) preparing a product for improving thyroid tissue lesions in a patient with thyroid disease;
(b5) preparing a product for improving the level of thyroid antibodies in a patient with thyroid disease;
(b6) preparing a product for reducing the level of reactive oxygen species (ROS) in a patient with thyroid disease;
(b7) preparing a product for improving the level of inflammatory factors in a patient with thyroid disease.

The product is a drug or a pharmaceutical preparation.

The thyroid disease comprises at least one of the following: hypothyroidism, hyperthyroidism, thyroid nodule, and thyroiditis.

The present disclosure also protects a method for preventing and/or treating and/or improving thyroid disease.

The method comprises the following step: administering the mulberry extract or its main active ingredient to a recipient animal or human to prevent and/or treat and/or improve thyroid disease.

In the present disclosure, the mulberry extract is mulberry branch extract, mulberry root bark extract, and/or mulberry leaf extract.

The mulberry extract acts on humans or mammals.

In one embodiment of the present disclosure, the mulberry extract mainly comprises alkaloids, and further comprises polysaccharides, flavonoids, and amino acids.

Preferably, the alkaloids comprise at least one of the following: 1-deoxynojirimycin (1-deoxynojirimycin or DNJ), N-methyl-1-deoxynojirimycin (N-methly-1-deoxynojirimycin), fagomine (fagomine or FAG), 3-epi-fagomine (3-epi-fagomine), 1,4-dideoxy-1,4-imino-D-arabinitol (1,4-dideoxy-1,4-imino-D-arabinitol or DAB), calystegin B2 (calysteginB2), calystegin C1 (calysteginC1), 2-O-(α-D-galactopyranosyl)-1-deoxynojirimycin (2-O-(α-D-galactopyranosyl)-1-deoxynojirimycin), 6-O-(β-D-glucopyranosyl)-1-deoxynoj irimycin (6-O-(β-D-glucopyranosyl)-1-deoxynojirimycin), and 1,4-dideoxy-1,4-imino-(2-O-β-D-glucopyranosyl)-D-arabinitol (1,4-dideoxy-1,4-imino-(2-O-β-D-glucopyranosyl)-D-arabinitol).

Preferably, the weight percentage of DNJ is not less than 50% of the total alkaloids (preferably 60-99%).

Preferably, the heavy metal content of the mulberry extract does not exceed 10 ppm.

Preferably, the mulberry extract comprises alkaloids in a weight content of more than 3% (preferably alkaloids in a weight content of 3-99%, more preferably alkaloids in a weight content of 15-99%, for example 15-90%, or 15-80%, or 15-75%, or 15-70%, further preferably alkaloids in a weight content of 30-99%, for example 30-90%, or 30-80%, or 30-75%, or 30-70%, or 35-70%, or 60-75%), and/or comprises polysaccharides in a weight content of not higher than 70% (preferably polysaccharides in a weight content of 0.2-50%, more preferably polysaccharides in a weight content of 0.2-35%, 0.2-25%, 0.2-23%, 20-25%, 5-35%, 3-35%, 1-35%, or preferably polysaccharides in a weight content of 20-70%, more preferably polysaccharides in a weight content of 20-50%, 20-40%, 20-35%, 20-30%, 20-23%), and/or comprises flavonoids in a weight content of not higher than 10% (preferably flavonoids in a weight content of 0.05-5%, more preferably flavonoids in a weight content of 0-7%, 0-2%, 0.05-2%, 0.5-1.5%, 0-1%, 0.05-1%), and/or comprises amino acids in a weight content of not higher than 50% (preferably amino acids in a weight content of 0-30%, more preferably amino acids in a weight content of 0-25%, 0-20%, 0-5%, 3-25% or 5-20%), and/or other components (the weight content is preferably 0-25%, more preferably 0-20%, 0-15%, 0-11%, 2-20%, 4-8%), the sum of the content of each component is 100%, wherein each component refers to all components in the plant extract including alkaloids, polysaccharides, flavonoids, and amino acids, that is, in the plant extract, in addition to alkaloids, polysaccharides, flavonoids, and amino acids, other components are also contained.

In the mulberry extract, the weight content of each component is as follows:
Alkaloids: 3-99%,
Polysaccharides: 0.2-70%,
Flavonoids: 0-10%,
Amino acids: 0-50%,
Other components: 0-25%;

Preferably, based on the mulberry extract, the weight content of each component is as follows:
Alkaloids: 30-99%,
Polysaccharides: 0.2-35%,
Flavonoids: 0-2%,
Amino acids: 0-30%,
Other components: 0-20%;

Preferably, based on the mulberry extract, the weight content of each component is as follows:
Alkaloids: 30-99%,
Polysaccharides: 0.2-35%,
Flavonoids: 0.05-2%,
Amino acids: 0-25%,
Other components: 0-20%;

Preferably, based on the mulberry extract, the weight content of each component is as follows:
Alkaloids: 40-99%,
Polysaccharides: 0.2-25%,
Flavonoids: 0.05-1%,
Amino acids: 0-25%,
Other components: 0-15%;

Preferably, based on the mulberry extract, the weight content of each component is as follows:
Alkaloids: 50-99%,
Polysaccharides: 0.2-23%,
Flavonoids: 0-1%,
Amino acids: 0-20%,
Other components: 0-11%;

Preferably, based on the mulberry extract, the weight content of each component is as follows:
Alkaloids: 50-99%,
Polysaccharides: 0.2-35%,
Flavonoids: 0-2%,
Amino acids: 0-30%,
Other components: 0-20%;

Preferably, based on the mulberry extract, the weight content of each component is as follows:
Alkaloids: 60-99%,
Polysaccharides: 0.2-23%,
Flavonoids: 0-1%,
Amino acids: 0-5%,
Other components: 0-11%;

Preferably, based on the mulberry extract, the weight content of each component is as follows:
Alkaloids: 63-99%,
Polysaccharides: 0.2-23%,
Flavonoids: 0-1%,
Amino acids: 0-5%,
Other components: 0-11%;

More preferably, based on the mulberry extract, the weight content of each component is as follows:
Alkaloids: 50-65%,
Polysaccharides: 20-25%,
Flavonoids: 0.5-1.5%,
Amino acids: 3-25%,
Other components: 2-20%;

More preferably, based on the mulberry extract, the weight content of each component is as follows:
Alkaloids: 50-65%,
Polysaccharides: 20-25%,
Flavonoids: 0.5-1.5%,
Amino acids: 5-20%,
Other components: 4-8%;

More preferably, based on the mulberry extract, the weight content of each component is as follows:
Alkaloids: 30-90%,
Polysaccharides: 0.2-35%,
Flavonoids: 0-2%,
Amino acids: 0-30%,
Other components: 0-20%;

Preferably, based on the mulberry extract, the weight content of each component is as follows:
Alkaloids: 30-90%,
Polysaccharides: 0.2-35%,
Flavonoids: 0.05-2%,
Amino acids: 0-25%,
Other components: 0-20%;

Preferably, based on the mulberry extract, the weight content of each component is as follows:
Alkaloids: 30-80%,
Polysaccharides: 0.2-35%,
Flavonoids: 0-2%,
Amino acids: 0-30%,
Other components: 0-11%;

Preferably, based on the mulberry extract, the weight content of each component is as follows:
Alkaloids: 30-80%,
Polysaccharides: 5-35%,
Flavonoids: 0.1-2%,
Amino acids: 4-25%,
Other components: 8-11%;

Preferably, based on the mulberry extract, the weight content of each component is as follows:
Alkaloids: 30-70%,
Polysaccharides: 0.2-35%,
Flavonoids: 0-2%,
Amino acids: 0-25%,
Other components: 0-8%;

Preferably, based on the mulberry extract, the weight content of each component is as follows:
Alkaloids: 30-70%,
Polysaccharides: 20-35%,
Flavonoids: 0.6-2%,
Amino acids: 5-25%,
Other components: 4-8%;

Preferably, based on the mulberry extract, the weight content of each component is as follows:
Alkaloids: 35-70%,
Polysaccharides: 0.2-35%,
Flavonoids: 0-2%,
Amino acids: 0-25%,
Other components: 0-8%;

Preferably, based on the mulberry extract, the weight content of each component is as follows:
Alkaloids: 35-70%,
Polysaccharides: 20-35%,
Flavonoids: 0.6-2%,
Amino acids: 5-25%,
Other components: 4-8%;

Preferably, based on the mulberry extract, the weight content of each component is as follows:
Alkaloids: 40-70%,
Polysaccharides: 0.2-25%,
Flavonoids: 0.05-1%,
Amino acids: 0-25%,
Other components: 0-10%;

Preferably, based on the mulberry extract, the weight content of each component is as follows:
Alkaloids: 40-70%,
Polysaccharides: 20-25%,
Flavonoids: 0.05-1%,
Amino acids: 5-25%,
Other components: 0-10%;

Preferably, based on the mulberry extract, the weight content of each component is as follows:
Alkaloids: 30-65%,
Polysaccharides: 0.2-35%,
Flavonoids: 0-2%,
Amino acids: 0-25%,
Other components: 0-8%;

Preferably, based on the mulberry extract, the weight content of each component is as follows:
Alkaloids: 30-65%,
Polysaccharides: 20-35%,
Flavonoids: 0.6-2%,
Amino acids: 5-25%,
Other components: 4-8%;

Preferably, based on the mulberry extract, the weight content of each component is as follows:
Alkaloids: 15-99%,
Polysaccharides: 0.2-20%,
Flavonoids: 0-10%,
Amino acids: 0-45%,
Other components: 0-15%;

Preferably, based on the mulberry extract, the weight content of each component is as follows:
Alkaloids: 15-90%,
Polysaccharides: 3-20%,
Flavonoids: 0-10%,
Amino acids: 0-45%,
Other components: 0-15%;

Preferably, based on the mulberry extract, the weight content of each component is as follows:
Alkaloids: 15-80%,
Polysaccharides: 5-35%,
Flavonoids: 0-10%,
Amino acids: 0-45%,
Other components: 0-15%;

Preferably, based on the mulberry extract, the weight content of each component is as follows:
Alkaloids: 15-70%,
Polysaccharides: 0.2-20%,
Flavonoids: 0-10%,
Amino acids: 0-45%,
Other components: 0-15%;

Preferably, based on the mulberry extract, the weight content of each component is as follows:
Alkaloids: 15-70%,
Polysaccharides: 0.2-20%,
Flavonoids: 0-10%,
Amino acids: 5-45%,
Other components: 0-15%;

Preferably, based on the mulberry extract, the weight content of each component is as follows:
Alkaloids: 15-65%,
Polysaccharides: 0.2-20%,
Flavonoids: 0-10%,
Amino acids: 0-45%,
Other components: 0-15%;

Preferably, based on the mulberry extract, the weight content of each component is as follows:
Alkaloids: 15-65%,
Polysaccharides: 0.2-20%,
Flavonoids: 0-10%,
Amino acids: 5-45%,
Other components: 0-15%;

In the mulberry extract, the weight content of each component is as follows:
Alkaloids: 15-30%,
Polysaccharides: 0.2-40%,
Flavonoids: 0-10%,
Amino acids: 20-50%,
Other components: 0-15%;

In the mulberry extract, the weight content of each component is as follows:
Alkaloids: 3-70%,
Polysaccharides: 0.2-70%,
Flavonoids: 0-10%,
Amino acids: 0-10%,
Other components: 0-10%;

In the mulberry extract, the weight content of each component is as follows:
Alkaloids: 3-70%,
Polysaccharides: 20-70%,
Flavonoids: 0-10%,
Amino acids: 5-10%,
Other components: 0-10%;

In the mulberry extract, the weight content of each component is as follows:
Alkaloids: 3-30%,
Polysaccharides: 35-70%,
Flavonoids: 0-10%,
Amino acids: 0-25%,
Other components: 0-10%;

In the mulberry extract, the weight content of each component is as follows:
Alkaloids: 3-30%,
Polysaccharides: 35-70%,
Flavonoids: 0-10%,
Amino acids: 0-25%,
Other components: 0-10%;

In the mulberry extract, the weight content of each component is as follows:
Alkaloids: 3-40%,
Polysaccharides: 25-70%,
Flavonoids: 0-10%,
Amino acids: 0-25%,
Other components: 0-10%;

In the mulberry extract, the weight content of each component is as follows:
Alkaloids: 3-40%,
Polysaccharides: 25-70%,
Flavonoids: 0-10%,
Amino acids: 10-25%,
Other components: 0-10%;

In the mulberry extract, the weight content of each component is as follows:
Alkaloids: 3-50%,
Polysaccharides: 20-70%,
Flavonoids: 0-10%,
Amino acids: 0-20%,
Other components: 0-10%;

In the mulberry extract, the weight content of each component is as follows:
Alkaloids: 3-50%,
Polysaccharides: 20-70%,
Flavonoids: 0-10%,
Amino acids: 10-20%,
Other components: 0-10%;

In the mulberry extract, the weight content of each component is as follows:
Alkaloids: 3-65%,
Polysaccharides: 20-70%,
Flavonoids: 0-10%,
Amino acids: 0-10%,
Other components: 0-10%;

In the mulberry extract, the weight content of each component is as follows:
Alkaloids: 3-65%,
Polysaccharides: 20-70%,
Flavonoids: 0-10%,
Amino acids: 5-10%,
Other components: 0-10%;

In the mulberry extract, the weight content of each component is as follows:
Alkaloids: 80-99%,
Polysaccharides: 0.2-10%,
Flavonoids: 0-1%,
Amino acids: 0-5%,
Other components: 0-15%.

In the present disclosure, the mulberry extract may be provided in the form of commercially available Mulberry Branch Total Alkaloids Tablets (National Medical Products Administration Approval Number Z20200002).

In one embodiment, the preparation method of the mulberry extract comprises the following steps: preparing a crude extract; optionally separating via cation resin and/or anion resin; optionally performing alcohol precipitation on the resin effluent; and optionally performing concentration and drying treatment. Alternatively, the mulberry extract may be prepared with reference to the method described in CN 110393738A. Preferably, the preparation method of the mulberry extract comprises the following steps: Step 1): preparing a crude extract; Step 2): separating via cation resin and/or optional anion resin; optional Step 3): performing alcohol precipitation on the resin effluent of Step 2); optional Step 4): concentration and drying treatment.

In the present disclosure, the preparation method of the mulberry extract comprises the following steps: 1) preparing a crude extract of a Moraceae plant; 2) separating the crude extract via cation resin and/or optional anion resin to obtain the mulberry extract. The method may further comprise the following steps: 3) performing alcohol precipitation on the resin effluent of Step 2) and collecting the supernatant; 4) performing concentration and drying treatment on the supernatant. The method may further comprise the following step: performing concentration and drying treatment on the resin effluent of Step 2). The Moraceae plant may be selected from Morus alba var. multicaulis, Morus alba var. rubra, Morus alba, Morus serrata, Morus mongolica, or hybrid mulberry, and the hybrid mulberry is preferably Yuesang No. 11, Guisangyou No. 62, or Sangteyou No. 2. Various parts of the plant such as leaves, roots, branches, bark, buds, stems, and fruits may be used.

In one embodiment, the mulberry extract is prepared according to the following steps: pulverizing mulberry branches, mulberry leaves, or mulberry root bark, performing heating reflux extraction with water and/or an alcohol solution or acid water, with the solvent amount being 3-20 times that of the raw medicinal material, repeating the extraction 1-3 times, combining the extracts, concentrating, loading onto a cation exchange resin, eluting with 0.2-3N ammonia water, concentrating the eluate, loading onto an anion exchange resin, collecting the non-adsorbed portion, adding ethanol, precipitating to remove impurities, and concentrating or drying to obtain the extract.

In one embodiment, the mulberry extract is prepared according to the following steps: pulverizing mulberry branches, mulberry leaves, or mulberry root bark, performing heating reflux extraction with water and/or an alcohol solution or acid water, with the solvent amount being 3-20 times that of the raw medicinal material, repeating the extraction 1-3 times, combining the extracts, concentrating, loading onto a cation exchange resin, eluting with 0.2-3N ammonia water, concentrating the eluate, loading onto an anion exchange resin, collecting the non-adsorbed portion, and concentrating or drying to obtain the extract.

In one embodiment, the mulberry extract is prepared according to the following steps: pulverizing mulberry branches, mulberry leaves, or mulberry root bark, performing heating reflux extraction with water and/or an alcohol solution or acid water, with the solvent amount being 3-20 times that of the raw medicinal material, repeating the extraction 1-3 times, combining the extracts, concentrating, loading onto a cation exchange resin, eluting with 0.2-3N ammonia water, and concentrating or drying the eluate to obtain the extract.

In one embodiment, the mulberry extract is prepared according to the following steps: pulverizing mulberry branches, mulberry leaves, or mulberry root bark, performing heating reflux extraction with water, with the solvent amount being 3-20 times that of the raw medicinal material (preferably 4-15 times, more preferably 4-12 times), repeating the extraction 1-3 times (the extraction time is preferably 0.5-3h each time, more preferably 1-2h each time), combining the extracts, concentrating, loading onto a cation exchange resin, eluting with 0.2-3N ammonia water, loading the eluate onto an anion exchange resin, collecting the non-adsorbed portion (i.e., the anion resin effluent), adding ethanol, precipitating to remove impurities, and concentrating or drying to obtain the extract.

Optionally, alcohol precipitation treatment may be performed on the concentrated crude extract before the resin separation treatment in Step 2). During the alcohol precipitation treatment, ethanol is added to the crude extract, stirred and mixed well, and then the stirring is stopped and the mixture is allowed to stand for a certain period of time to precipitate the insolubles. Preferably, calculated in L/kg, the volume-to-mass ratio of the added ethanol to the plant raw material is 0.2-20, preferably 0.4-10 times. More preferably, an alcohol precipitation tank is used for the alcohol precipitation treatment. Preferably, the stirring speed in the alcohol precipitation treatment is 10-600 rpm, preferably 40-500 rpm, more preferably 80-400 rpm or 300 rpm.

Preferably, after packing the cation resin column, activation is performed in the order of acidic solution wash, alkaline solution wash, and acidic solution wash. Preferably, the alkaline solution wash is continued until the pH of the effluent is 8.0-9.5, preferably 8.5-9.5; preferably, the alkaline solution is selected from ammonia water, sodium hydroxide solution, potassium hydroxide solution, or sodium carbonate solution; preferably, the concentration of the alkaline solution is 0.5-4 mol/L, preferably 1-2 mol/L. Preferably, the acidic solution wash is continued until the pH of the effluent is 3.0-7.0, preferably 4.5-6.5. Preferably, the acidic solution is selected from hydrochloric acid solution, phosphoric acid solution, or disodium hydrogen phosphate-citric acid buffer; preferably, the concentration of the acidic solution is 0.5-4 mol/L, preferably 1.5-2 mol/L. Optionally, after the final acidic solution wash, the cation resin may be rinsed with 3-5 times the column volume of deionized water.

Preferably, the cation resin is 732 type strong acid styrene series cation exchange resin, 002SC type strong acid styrene series cation resin, 734 type strong acid styrene series cation exchange resin, D001 type macroporous strong acid styrene series cation exchange resin, or D113 type macroporous weak acid cation resin.

Preferably, the weight ratio of the amount of the cation resin to the plant raw material feed is 1:1-30 (preferably 1:1-25, 1:2-20, 1:2-15, 1:2-10, 1:2-7, 1:2-3). After loading the plant crude extract onto the cation resin, the loaded cation resin is eluted with an eluent. Preferably, the eluent is a salt solution containing cations or an alkaline solution, preferably one or more of sodium chloride, ammonium chloride, ammonium sulfate, ammonium nitrate, ammonia water, potassium chloride, and sodium hydroxide. Preferably, the concentration of the eluent is 0.04-5 mol/L (preferably 0.5-2.5 mol/L, 0.2-3 mol/L, more preferably 0.5-2.5 mol/L). Preferably, the eluent flow rate is 1-15 BV/h (preferably 5-10 BV/h, more preferably 5-6 BV/h). The starting point of collection may be determined based on the pH of the cation resin effluent; for example, when eluting with an alkaline solution such as ammonia water, the eluate is collected when the pH of the cation column effluent is >7, or the starting point of effluent collection may be determined based on color development or precipitation reaction; preferably, when the volume of the collected liquid reaches 0.1-10 times (more preferably 0.2-5 times) the weight of the plant raw material feed, collection is stopped, and the collected liquid is optionally purified by passing through an anion column.

When purifying by passing through an anion column, preferably, after packing the anion resin column, activation is performed in the order of alkaline solution wash, acidic solution wash, and alkaline solution wash. Preferably, the alkaline solution wash is continued until the pH of the effluent is 8.0-9.5, preferably 8.5-9.5; preferably, the alkaline solution is selected from ammonia water, sodium hydroxide solution, potassium hydroxide solution, or sodium carbonate solution; preferably, the concentration of the alkaline solution is 0.5-4 mol/L, preferably 1-2 mol/L. Preferably, the acidic solution wash is continued until the pH of the effluent is 3.0-7.0, preferably 4.5-6.5. Preferably, the acidic solution is selected from hydrochloric acid solution, phosphoric acid solution, or disodium hydrogen phosphate-citric acid buffer; preferably, the concentration of the acidic solution is 0.5-4 mol/L, preferably 1-2 mol/L.

Preferably, the anion resin is 711 type strong base styrene series anion resin, 717 type strong base styrene series anion exchange resin, D201 type macroporous strong base styrene series anion exchange resin, or D218 type macroporous strong base acrylic series anion exchange resin. Preferably, the weight ratio of the amount of the anion resin to the plant raw material feed is 1:1-80 (preferably 1:1-64, 1:1-32, 1:1-24, 1:5-16, 1:3). Collection starts when the liquid flows out of the anion resin. Preferably, when the volume of the collected liquid reaches 0.05-10 times (preferably 0.1-5 times) the weight of the plant raw material feed, collection is stopped.

Preferably, the weight ratio of ethanol used in the alcohol precipitation treatment to the plant raw material feed is 1:4-600 (preferably 1:20-300, more preferably 1:20-50, 1:40, 1:80, 1:22). In the alcohol precipitation treatment, the stirring speed is 10-600 rpm (preferably 40-500 rpm, 80-400 rpm). The alcohol precipitation treatment time is 12-24h.

Furthermore, before the alcohol precipitation treatment, the method further comprises the steps of performing centrifugation or microfiltration membrane filtration to remove impurities from the anion resin effluent, followed by reverse osmosis membrane concentration. The specific gravity of the concentrated liquid may be 1.0-1.3, preferably 1.1-1.25.

Preferably, the drug further comprises a pharmaceutically acceptable carrier. The carrier is an inactive component that is non-toxic to the human body and is suitable for the route of administration or mode of administration. The carrier may be a solid or liquid excipient. Solid excipients include, for example, microcrystalline cellulose, mannitol, lactose, pregelatinized starch, low-substituted hydroxypropyl cellulose, crospovidone, sodium carboxymethyl starch, aspartame, calcium hydrogen phosphate, sodium lactate, poloxamer, sodium dodecyl sulfate, sodium carboxymethyl cellulose, gelatin, xanthan gum, povidone, starch, magnesium stearate, sodium carboxymethyl starch, and talc; liquid excipients include, for example, water, ethanol, syrup, and glycerin.

Preferably, the drug is an oral dosage form; more preferably, the drug is a tablet, capsule, oral solution, oral emulsion, pill, granule, syrup, or powder.

### Beneficial Effects

The present disclosure has been proven by pharmacodynamic experiments that: the mulberry extract can regulate the secretion levels of TSH, FT3, and FT4 in patients with thyroid disease, regulate the levels of thyroid antibodies in patients with Hashimoto's thyroiditis, has a significant improving effect on thyroid tissue lesions, and can be used for treating thyroid diseases.

### BRIEF DESCRIPTION OF THE DRAWINGS

In order to more clearly illustrate the specific embodiments of the present disclosure or the technical solutions in the prior art, the following provides a brief introduction to the drawings that need to be used in the description of the specific embodiments or the prior art. It is obvious that the drawings in the following description are some embodiments of the present disclosure, and for those of ordinary skill in the art, other drawings can also be obtained from these drawings without creative work.
FIG. 1 shows the levels of serum thyroid-stimulating hormone (TSH), free triiodothyronine (FT3), and free thyroxine (FT4) in rats from the blank group, model group, and administration group after 21 days of administration in Experimental Example 1. * indicates P < 0.05 compared with the model group, and ** indicates P < 0.01.
FIG. 2 is a photograph of HE staining of rat thyroid tissue from the blank group, model group, and administration group after 21 days of administration in Experimental Example 1.
FIG. 3 shows the levels of serum thyroid-stimulating hormone (TSH), free triiodothyronine (FT3), and free thyroxine (FT4) in rats from the blank group, model group, and administration group after 21 days of administration in Experimental Example 2. * indicates P < 0.05 compared with the model group, and ** indicates P < 0.01.
FIG. 4 is a photograph of HE staining of rat thyroid tissue from the blank group, model group, and administration group after 21 days of administration in Experimental Example 2.
FIG. 5 shows the results of serum antibody determination in rats from the blank group, model group, and administration group after 21 days of administration in Experimental Example 3.
FIG. 6 is a photograph of HE staining of rat thyroid tissue from the blank group, model group, and administration group after 21 days of administration in Experimental Example 3.
FIG. 7 shows the levels of anti-thyroid peroxidase antibody and anti-thyroglobulin antibody in four patients with Hashimoto's thyroiditis before and after medication in the human experiment of Example 4.
FIG. 8 shows the content of TNF-α in the supernatant of each group in the thyroiditis cell modeling experiment of Example 4. * indicates P < 0.05 compared with the model group, ** indicates P < 0.01, and *** indicates P < 0.001.
FIG. 9 shows the content of FT4 in the supernatant of each group in the thyroiditis cell modeling experiment of Example 4. * indicates P < 0.05 compared with the model group, ** indicates P < 0.01, and *** indicates P < 0.001.
FIG. 10 shows the content of IL-6 in the supernatant of each group in the thyroiditis cell modeling experiment of Example 4. * indicates P < 0.05 compared with the model group, and ** indicates P < 0.01.

### DETAILED DESCRIPTION

The present disclosure is further described in detail below through examples. Through these exemplary descriptions, the features and advantages of the present disclosure will become clearer and more explicit. However, the present disclosure is not limited to the following examples. Unless otherwise specified, the methods described are all conventional methods. Unless otherwise specified, the raw materials can all be obtained from public commercial sources.

Herein, the professional term "exemplary" means "used as an example, embodiment, or illustration." Any embodiment described herein as "exemplary" is not necessarily to be construed as superior or better than other embodiments.

Furthermore, the technical features involved in different embodiments of the present disclosure described below may be combined with each other as long as they do not conflict with each other. In this specification, different component ranges can be arbitrarily combined; the upper and lower limit values of the numerical ranges indicated by "above," "below," "~," or "-" are values that can be arbitrarily combined. Furthermore, the numerical values from examples (including preparation examples and experimental examples) can also be used as the upper and lower limit values.

The component levels involved in the present disclosure are detected according to publicly disclosed methods (referring to the methods described in the patents with publication numbers CN111077247A and CN110393738A).

### I. Preparation Examples of Mulberry Extract

### Preparation Example 1 of Mulberry Extract

1000 kg of fresh mulberry branches (Morus serrata Yuesang No. 11) is taken, pulverized, 4000 L of water is added, and extracted by heating reflux for 2 hours. The extracts are combined and filtered to remove insolubles to obtain a crude extract. The crude extract is hot-concentrated until the mass percentage of solids reaches 4%, and maintained at 50°C to serve as the loading solution for the cation resin column.

It is achieved by packing a column with 150 kg of D113-type macroporous weak acid styrene-acrylic series cation exchange resin. The resin is then washed sequentially with a 2 mol/L hydrochloric acid solution until the effluent pH reaches 4.5, followed by a 1 mol/L sodium hydroxide solution until the effluent pH reaches 8.5, and again with a 2 mol/L hydrochloric acid solution until the effluent pH stabilizes at 4.5. Subsequently, rinsing is performed with 5 times the column volume of deionized water to complete activation. After loading the concentrated extract, elution is carried out using 1000 L of 2.5 mol/L ammonia water at a flow rate of 6 BV/h. Collection of the eluate is initiated when the pH of the cation column effluent exceeds 7 and terminated once the collected volume reaches 900 L. The collected solution is then directly passed through an anion column for further purification.

It is achieved by packing a column with 62.5 kg of D218-type macroporous strong base acrylic series anion exchange resin. The resin is then washed sequentially with a 1.5 mol/L sodium hydroxide solution until the effluent pH reaches 9.0, followed by a 1.5 mol/L hydrochloric acid solution until the effluent pH drops to 3.5, and again with a 1.5 mol/L sodium hydroxide solution until the effluent pH stabilizes at 9.0, thereby completing activation. Subsequently, the collected cation resin eluate is loaded onto the anion resin column, and effluent collection is continued until the total volume reaches 920 L.

It is achieved by subjecting the collected liquid to centrifugation to remove impurities, followed by concentration using a reverse osmosis membrane until the specific gravity of the concentrated liquid reaches 1.25. The concentrated liquid is then transferred to an alcohol precipitation tank, to which 25 L of anhydrous ethanol is added under stirring at 500 rpm using a paddle mixer. After complete addition of ethanol, stirring is discontinued, and alcohol precipitation is allowed to proceed for 24 hours. The supernatant is subsequently collected and concentrated under reduced pressure to yield mulberry branch extract paste (designated as mulberry extract SZ-A).

In the mulberry branch extract paste, the mass percentage of alkaloids is 52% (the mass percentage of DNJ in the alkaloids is 65%), the mass percentage of polysaccharides is 22%, the mass percentage of flavonoids is 0.8%, and the mass percentage of amino acids is 20%.

### Preparation Example 2 of Mulberry Extract

It is conducted by processing 10 kg of fresh mulberry branches (Sangteyou No. 2) through pulverization, followed by two-stage water addition (150 L total) and decoction extraction for 3 hours per stage. The combined extracts are filtered to remove insolubles, then hot-concentrated until the solid mass percentage reaches 8%. The concentrated extract is transferred to an alcohol precipitation tank, to which 2,367.9 g (3 L) of anhydrous ethanol is added under paddle stirring at 300 rpm. After complete ethanol addition, stirring is discontinued, and alcohol precipitation is maintained for 24 hours. The resulting supernatant serves as the loading solution for cation resin chromatography. A column is packed with 5 kg of 002SC-type strong acid styrene series cation resin, which is activated according to the method described in Example 1. The alcohol-precipitated extract is loaded onto the activated resin column, followed by elution with 100 L of 5 mol/L potassium chloride solution at a flow rate of 5 BV/h."**Detection of the effluent is performed using 20% silicotungstic acid solution. Collection is initiated upon formation of a white precipitate and terminated when the accumulated volume reaches 25 L. The collected liquid is subsequently passed directly through an anion exchange column for purification.

Column packing is performed with 10 kg of 711-type strong base styrene series anion resin, which is activated according to the method described in Example 1. The collected cation resin eluate is loaded onto the activated anion resin column, and effluent collection is continued until the accumulated volume reaches 15 L. The collected liquid is then reloaded onto the cation resin column, and the sequential separation process using cation and anion resins is repeated twice more following the aforementioned procedure.

It is performed that the collected liquid obtained after three column separations undergoes centrifugation to remove impurities, followed by concentration using a reverse osmosis membrane until a specific gravity of 1.25 is achieved. Subsequently, the concentrated liquid is transferred to an alcohol precipitation tank, where 125 g of anhydrous ethanol is added under stirring with a paddle at 1000 rpm. After ethanol addition, stirring is ceased, and alcohol precipitation is carried out for 24 hours. The supernatant is then collected and concentrated under reduced pressure to obtain the extract paste. Additionally, it is conducted that fresh mulberry root bark and mulberry leaves (Sangteyou No. 2) are extracted using the identical method and parameters as described above.

In the obtained mulberry branch extract, the mass percentage of alkaloids is 98%, polysaccharides is 0.2%, flavonoids is 0.05%, and amino acids is 0%. In the alkaloids, the content of 1-DNJ is 99%.

In the obtained mulberry root bark extract, the mass percentage of alkaloids is 95%, polysaccharides is 2%, flavonoids is 0.1%, and amino acids is 1%. In the alkaloids, the content of 1-DNJ is 96%.

In the obtained mulberry leaf extract, the mass percentage of alkaloids is 90%, polysaccharides is 4%, flavonoids is 0.1%, and amino acids is 3%. In the alkaloids, the content of 1-DNJ is 91%.

### Preparation Example 3 of Mulberry Extract

It is conducted that 1000 kg of fresh mulberry branches (Morus alba var. multicaulis) are pulverized, followed by the addition of 11,500 L of water and heating reflux extraction for 2 hours. The extracts are combined and filtered to remove insolubles, yielding a crude extract. Subsequently, it is performed that the crude extract undergoes centrifugation to eliminate impurities, followed by concentration via reverse osmosis membrane until the solid mass percentage reaches 1%, serving as the loading solution for the cation resin column.

It is carried out that a column is packed with 300 kg of D001-type macroporous strong acid styrene-series cation exchange resin, and the resin is activated according to the method described in Preparation Example 1. The concentrated crude extract is loaded onto the column, followed by elution with 5,000 L of 0.04 mol/L ammonium nitrate at an elution rate of 5 BV/h. The effluent is monitored using 20% silicotungstic acid; collection is initiated upon the formation of a white precipitate and terminated when the collected volume reaches 1,000 L.

The collected liquid obtained after cation column separation is concentrated by a nanofiltration membrane and then concentrated under reduced pressure to obtain the extract paste.

In the obtained mulberry branch extract, the mass percentage of alkaloids is 15%, polysaccharides is 20%, flavonoids is 7%, and amino acids is 45%. In the alkaloids, the content of 1-DNJ is 55%.

### Preparation Example 4 of Mulberry Extract

It is conducted that 333 kg of dried mulberry branches (Yuesang No. 11) are pulverized, followed by the addition of 4,000 L of water and two rounds of heating reflux extraction, each lasting 1 hour. The extracts are combined, filtered, and concentrated to a density of 1 kg crude drug/L.

It is performed that a column is packed with 150 kg of D113-type macroporous weak acid styrene-acrylic series cation exchange resin. The resin is activated by sequential washing with: (1) a 2 mol/L hydrochloric acid solution until the effluent pH reaches 4.5; (2) a 1 mol/L sodium hydroxide solution until pH 8.5; (3) a 2 mol/L hydrochloric acid solution until pH 4.5; and finally rinsed with 5 column volumes (CV) of deionized water. The concentrated extract is loaded onto the activated column and eluted with 1,000 L of 2.5 mol/L ammonia solution at an elution rate of 6 BV/h. Eluate collection is initiated when the cation column effluent pH exceeds 7 and terminated upon reaching 900 L of collected liquid, which is then directly passed through an anion column for purification.

It is carried out that a column is packed with 125 kg of D218-type macroporous strong base acrylic series anion exchange resin. Activation is achieved by sequential washing with: (1) a 1.5 mol/L sodium hydroxide solution until effluent pH 9.0; (2) a 1.5 mol/L hydrochloric acid solution until pH 3.5; (3) a 1.5 mol/L sodium hydroxide solution until pH 9.0. The cation resin eluate is loaded onto the activated anion column, and effluent with pH >8 is collected until the volume reaches 870 L.

The collected liquid from anion column separation is filtered through a microfiltration membrane to remove impurities, followed by concentration via reverse osmosis membrane to a specific gravity of 1.1. The concentrate is transferred to an alcohol precipitation tank, where 15 kg of anhydrous ethanol is added under stirring at 400 rpm using a paddle mixer. After ethanol addition, stirring is ceased, and alcohol precipitation is conducted for 24 hours. The supernatant is collected and concentrated under reduced pressure to obtain mulberry branch extract paste. The final product composition is as follows: alkaloids (80% w/w, including 75% 1-DNJ), polysaccharides (5%), flavonoids (0.1%), and amino acids (4%).

### Preparation Example 5 of Mulberry Extract

It is carried out that 400 kg of dried mulberry branches (Yuesang No. 11) are pulverized, followed by the addition of 4,000 L of water and two rounds of heating reflux extraction, each lasting 1 hour. The extracts are combined, filtered, and concentrated to a density of 1 kg crude drug/L.

It is performed that a column is packed with 62.5 kg of D218-type macroporous strong base acrylic series anion exchange resin. The resin is activated by sequential washing with: (1) a 1.5 mol/L sodium hydroxide solution until the effluent pH reaches 9.0; (2) a 1.5 mol/L hydrochloric acid solution until pH 3.5; and (3) a 1.5 mol/L sodium hydroxide solution until pH 9.0. The collected and concentrated extract is loaded onto the activated anion resin, and the effluent is collected thereafter.

The collected liquid obtained after anion column separation is filtered through a microfiltration membrane to remove impurities and then concentrated by reverse osmosis membrane, followed by further concentration under reduced pressure and drying to obtain mulberry branch extract paste. Sample content: the mass percentage of alkaloids is 3%, polysaccharides is 70%, flavonoids is 10%, and amino acids is 10%. In the alkaloids, the content of 1-DNJ is 68%.

### Preparation Example 6 of Mulberry Extract

It is required that 1500 kg of fresh mulberry branches (Morus serrata Yuesang No. 11) be taken and pulverized, after which 6,000 L of water be added. Next, it is performed that a heating reflux extraction be carried out on the mixture for 2 hours. After that, it is conducted that the extracts be combined and filtered to remove insoluble substances, resulting in a crude extract being obtained. Subsequently, it is ensured that the crude extract be concentrated under heat until the mass percentage of solids reaches 4%, and it be maintained at 50°C for use as the loading solution for the cation resin column.

A column packing is confirmed with 100 kg of D113-type macroporous weak acid styrene-acrylic cation exchange resin. It is conducted that the resin be washed sequentially with: a 2 mol/L hydrochloric acid solution until the effluent pH is adjusted to 4.5; a 1 mol/L sodium hydroxide solution until the effluent pH is raised to 8.5; a 2 mol/L hydrochloric acid solution again until the effluent pH is lowered to 4.5. It is then required that the column be rinsed with 5 column volumes of deionized water to complete activation. It is specified that the concentrated extract be loaded onto the activated cation column, followed by elution with 1,000 L of 2.5 mol/L ammonia water at a flow rate of 6 BV/h. It is determined that eluate collection be initiated when the effluent pH from the cation column is detected to exceed 7, and be terminated when the collected volume reaches 900 L. It is directed that the collected liquid be immediately transferred through an anion exchange column for purification.

It is performed that a column be packed with 62.5 kg of D218-type macroporous strong base acrylic anion exchange resin. It is conducted that the resin be washed sequentially with: a 1.5 mol/L sodium hydroxide solution until the effluent pH is adjusted to 9.0; a 1.5 mol/L hydrochloric acid solution until the effluent pH is reduced to 3.5; a 1.5 mol/L sodium hydroxide solution again until the effluent pH is restored to 9.0 to complete activation. It is specified that the cation resin eluate be loaded onto the activated anion column, and it is required that effluent collection be continued until the volume reaches 870 L. It is reported that the collected effluent be concentrated under reduced pressure to obtain mulberry branch extract paste, in which: the mass percentage of alkaloids is 30% (with 1-DNJ content at 62%), polysaccharides constitute 35%, flavonoids account for 2%, amino acids make up 25%.

### Preparation Example 7 of Mulberry Extract

It is initiated that 1,000 kg of fresh mulberry branches (Morus serrata Yuesang No. 11) be taken and pulverized. It is then conducted that 4,000 L of water be added, followed by heating reflux extraction for 2 hours. It is required that the extracts be combined and filtered to remove insolubles, yielding a crude extract. It is specified that the crude extract be hot-concentrated until the mass percentage of solids reaches 4%, and maintained at 50°C to serve as the loading solution for the cation resin column.

It is performed that a column be packed with 100 kg of D113-type macroporous weak acid styrene-acrylic cation exchange resin. It is conducted that the resin be washed sequentially with: a 2 mol/L hydrochloric acid solution until the effluent pH is adjusted to 4.5; a 1 mol/L sodium hydroxide solution until the effluent pH is raised to 8.5; a 2 mol/L hydrochloric acid solution again until the effluent pH is lowered to 4.5. It is then required that the column be rinsed with 5 column volumes of deionized water to complete activation. It is specified that the concentrated extract be loaded onto the activated cation column, followed by elution with 1,000 L of 2.5 mol/L ammonia water at a flow rate of 6 BV/h. It is determined that eluate collection be initiated when the effluent pH from the cation column is detected to exceed 7, and be terminated when the collected volume reaches 900 L. It is directed that the collected liquid be immediately transferred through an anion column for purification.

It is performed that a column be packed with 62.5 kg of D218-type macroporous strong base acrylic anion exchange resin. It is conducted that the resin be washed sequentially with: a 1.5 mol/L sodium hydroxide solution until the effluent pH is adjusted to 9.0; a 1.5 mol/L hydrochloric acid solution until the effluent pH is reduced to 3.5; a 1.5 mol/L sodium hydroxide solution again until the effluent pH is restored to 9.0 to complete activation. It is specified that the cation resin eluate be loaded onto the activated anion column, and it is required that effluent collection be continued until the volume reaches 870 L. It is reported that the collected effluent be concentrated under reduced pressure to obtain mulberry branch extract paste, in which: the mass percentage of alkaloids is 40% (with 1-DNJ content at 57%), polysaccharides constitute 25%, flavonoids account for 0.5%, amino acids make up 25%.

### Preparation Example 8 of Mulberry Extract

It is specified that 333 kg of dried mulberry branches (Yuesang No. 11) be taken and pulverized. It is then conducted that 4,000 L of water be added, followed by heating reflux extraction twice, with each reflux lasting for 1 hour. It is required that the extracts be combined, filtered, and concentrated to a density of 1 kg crude drug/L.

It is performed that a column be packed with 150 kg of D113-type macroporous weak acid styrene-acrylic cation exchange resin. It is conducted that the resin be washed sequentially with: a 2 mol/L hydrochloric acid solution until the effluent pH is adjusted to 4.5; a 1 mol/L sodium hydroxide solution until the effluent pH is raised to 8.5; a 2 mol/L hydrochloric acid solution again until the effluent pH is lowered to 4.5. It is then required that the column be rinsed with 5 column volumes of deionized water to complete activation. It is specified that the concentrated extract be loaded onto the activated cation column, followed by elution with 1,000 L of 2.5 mol/L ammonia water at a flow rate of 6 BV/h. It is determined that eluate collection be initiated when the effluent pH from the cation column is detected to exceed 7, and be terminated when the collected volume reaches 900 L. It is directed that the collected liquid be immediately transferred through an anion column for purification.

It is performed that a column be packed with 62.5 kg of D218-type macroporous strong base acrylic anion exchange resin. It is conducted that the resin be washed sequentially with: a 1.5 mol/L sodium hydroxide solution until the effluent pH is adjusted to 9.0; a 1.5 mol/L hydrochloric acid solution until the effluent pH is reduced to 3.5; a 1.5 mol/L sodium hydroxide solution again until the effluent pH is restored to 9.0 to complete activation. It is specified that the cation resin eluate be loaded onto the activated anion column, and it is required that effluent collection be continued until the volume reaches 870 L, with the effluent pH maintained above 8.

It is reported that the collected liquid from the anion column be filtered through a microfiltration membrane to remove impurities, followed by concentration via reverse osmosis membrane until the specific gravity reaches 1.1. It is then directed that the concentrated liquid be transferred to an alcohol precipitation tank, where 15 kg of anhydrous ethanol be added under stirring at 400 rpm using a paddle mixer. It is specified that stirring be stopped after ethanol addition, followed by alcohol precipitation for 24 hours. It is required that the supernatant be collected and concentrated under reduced pressure to obtain mulberry branch extract paste, in which: the mass percentage of alkaloids is 63% (with 1-DNJ content at 61.9%), Polysaccharides constitute 23%, Flavonoids account for 1%, Amino acids make up 5%.

### Preparation Example 9 of Mulberry Extract

It is specified that 1,000 kg of fresh mulberry branches (Yuesang No. 11) be taken and pulverized. It is then conducted that 4,000 L of water be added, followed by heating reflux extraction for 2 hours. It is required that the extracts be combined and filtered to remove insolubles, resulting in a crude extract. It is performed that the crude extract be hot-concentrated until the solid content reaches 4%, and be maintained at 50°C to serve as the loading solution for the cation resin column.

It is performed that a column be packed with 120 kg of D113-type macroporous weak acid styrene-acrylic cation exchange resin. It is conducted that the resin be washed sequentially with: a 2 mol/L hydrochloric acid solution until the effluent pH is adjusted to 4.5; a 1 mol/L sodium hydroxide solution until the effluent pH is raised to 8.5; a 2 mol/L hydrochloric acid solution again until the effluent pH is lowered to 4.5. It is then required that the column be rinsed with 5 column volumes of deionized water to complete activation. It is specified that the concentrated extract be loaded onto the activated cation column, followed by elution with 1,000 L of 2.5 mol/L ammonia water at a flow rate of 6 BV/h. It is determined that eluate collection be initiated when the effluent pH from the cation column is detected to exceed 7, and be terminated when the collected volume reaches 900 L. It is directed that the collected liquid be immediately transferred through an anion column for purification.

It is performed that a column be packed with 45 kg of D218-type macroporous strong base acrylic anion exchange resin. It is conducted that the resin be washed sequentially with: a 1.5 mol/L sodium hydroxide solution until the effluent pH is adjusted to 9.0; a 1.5 mol/L hydrochloric acid solution until the effluent pH is reduced to 3.5; a 1.5 mol/L sodium hydroxide solution again until the effluent pH is restored to 9.0 to complete activation. It is specified that the cation resin eluate be loaded onto the activated anion column, and it is required that effluent collection be continued until the volume reaches 870 L.

The collected liquid from the anion column be filtered through a microfiltration membrane to remove impurities, followed by concentration via reverse osmosis membrane until the specific gravity reaches 1.1. It is then directed that the concentrated liquid be transferred to an alcohol precipitation tank, where 15 kg of anhydrous ethanol be added under stirring at 300 rpm using a paddle mixer. It is specified that stirring be stopped after ethanol addition, followed by alcohol precipitation for 24 hours. It is required that the supernatant be collected and concentrated under reduced pressure to obtain the extract paste, in which: the mass percentage of alkaloids is 70% (comprising 70% 1-DNJ, 13% FAG, and 10% DAB), polysaccharides constitute 20%, flavonoids account for 0.6%, amino acids make up 5%.

In the alkaloids, the content of 1-DNJ is 70%, FAG is 13%, and DAB is 10%.

### Preparation Example 10 of Mulberry Extract

It is specified that 80 kg of fresh mulberry branches (Guisangyou No. 62) be taken and pulverized. It is conducted that 1,000 L of water be added, followed by heating reflux extraction for 2 hours. It is required that the extracts be combined and filtered to remove insolubles, resulting in a crude extract. It is performed that the crude extract be hot-concentrated until the solid content reaches 4%, and be maintained at 50°C to serve as the loading solution for the cation resin column.

It is performed that a column be packed with 50 kg of D113-type macroporous weak acid styrene-acrylic cation exchange resin. It is conducted that the resin be washed sequentially with: a 2 mol/L hydrochloric acid solution until the effluent pH is adjusted to 4.5; a 1 mol/L sodium hydroxide solution until the effluent pH is raised to 8.5; a 2 mol/L hydrochloric acid solution again until the effluent pH is lowered to 4.5. It is then required that the column be rinsed with 5 column volumes of deionized water to complete activation. It is specified that the concentrated extract be loaded onto the activated cation column, followed by elution with 600 L of 2.5 mol/L ammonia water at a flow rate of 6 BV/h. It is determined that eluate collection be initiated when the effluent pH from the cation column is detected to exceed 7, and be terminated when the collected volume reaches 800 L. It is directed that the collected liquid be immediately transferred through an anion column for purification.

It is performed that a column be packed with 65 kg of D218-type macroporous strong base acrylic anion exchange resin. It is conducted that the resin be washed sequentially with: a 1.5 mol/L sodium hydroxide solution until the effluent pH is adjusted to 9.0; a 1.5 mol/L hydrochloric acid solution until the effluent pH is reduced to 3.5; a 1.5 mol/L sodium hydroxide solution again until the effluent pH is restored to 9.0 to complete activation. It is specified that the cation resin eluate be loaded onto the activated anion column, and it is required that effluent collection be continued until the volume reaches 750 L. It is further directed that the collected liquid be reloaded onto the cation resin column, and it is conducted that secondary separation be performed using cation and anion resins in sequence according to the aforementioned method.

It is reported that the collected liquid from the secondary separation be filtered through a microfiltration membrane to remove impurities, followed by concentration via reverse osmosis membrane until the specific gravity reaches 1.1. It is then directed that the concentrated liquid be transferred to an alcohol precipitation tank, where 400 g of anhydrous ethanol be added under stirring at 350 rpm using a paddle mixer. It is specified that stirring be stopped after ethanol addition, followed by alcohol precipitation for 24 hours. It is required that the supernatant be collected and concentrated under reduced pressure to obtain the extract paste, in which: the mass percentage of alkaloids is 90% (comprising 90% alkaloids, 3% polysaccharides, 0.5% flavonoids, and 2% amino acids), polysaccharides constitute 3%, flavonoids account for 0.5%, amino acids make up 2%.

In the alkaloids, the content of 1-DNJ is 94%, FAG is 2%, and DAB is 1%.

### II. Efficacy Verification of Mulberry Extract

### Experimental Example 1

### 1.Experimental Animals

Thirty 3-week-old, newly weaned, healthy female Sprague-Dawley (SD) rats with glossy coats are selected and housed under Specific Pathogen-Free (SPF) conditions.

### 2. Experimental Drugs

1% CMC: 1 g of CMC is weighed and added to 100 mL of pure water. The mixture is placed on a magnetic stirrer at 60°C and stirred at 500 r/min until the CMC is completely dissolved and the solution is transparent.

Letrozole-1% CMC solution (LZ): 0.25 mg/mL. A certain amount of letrozole (LZ) is weighed, dissolved in 1% CMC solution, and stirred to mix well.

Mulberry extract SZ-A prepared in Preparation Example 1: This is formulated into different concentrations and dissolved in an aqueous solution.

### 3. Modeling and Administration

The hypothalamic-pituitary-thyroid (HPT) axis and the hypothalamic-pituitary-ovarian (HPO) axis form a two-way loop. An abnormality in one axis can affect the normal function of the other. When the body is exposed to exogenous androgens in excess or given an inducer that inhibits androgen metabolism, leading to high androgen levels, the HPO axis is disrupted, which can cause hyperandrogenemia or Polycystic Ovary Syndrome (PCOS). This, in turn, affects the pituitary and thyroid functions regulated by the hypothalamus, leading to abnormal levels of thyroid-stimulating hormone (TSH) secreted by the pituitary or FT3 and FT4 secreted by the thyroid. Consequently, the body may develop various symptoms such as hyperthyroidism or hypothyroidism, as well as thyroid inflammation or nodules.

Thirty 3-week-old female SD rats in good health with glossy coats are selected. After 3 days of adaptive feeding, they are randomly divided into a blank group, a model group, and a treatment group. The rats in the blank group are fed a standard chow diet. The model and treatment groups are fed a high-fat diet (HFD, Research Diets D12492) for 3 weeks, followed by intragastric administration of LZ (1 mg/kg/day) for 6 weeks while continuing the HFD to establish the rat model. After the model is successfully evaluated (compared with the Blank group, the Model group showed a significant decrease in serum thyroid-stimulating hormone (TSH) and a significant increase in free triiodothyronine (FT3) and free thyroxine (FT4)), the treatment group is administered the drug according to the dosage and method in Table 1. During the administration period, the blank and model groups received an equivalent volume of normal saline. After 21 days of treatment, samples are collected for efficacy evaluation.

**Table 1**

| Group | Abbrevia -tion | Number of Animals | Dosage (calculated as total mulberry branch alkaloids, mg/kg/day) | | Route of Administration | Diet |
|---|---|---|---|---|---|---|
| Blank Group | Blank | 6 | 0 | | | Chow |
| Model Group | Model | 6 | 0 | | | HFD |
| Treatment Group | SZ-A-L | 6 | 67 | | Oral gavage | |
| Treatment Group | SZ-A-M | 6 | 100 | | | |
| Treatment Group | SZ-A-H | 6 | 150 | | | |

### 4.Observation Indicators and Detection Methods

(1) Expression of related hormones in serum: On the 21st day after administration, serum FT3, FT4, and TSH levels are measured in all groups of rats.
   Serum FT3, FT4, and TSH are all detected using ELISA. The kits are purchased from:
   1) Free Thyroxine (FT4) ELISA Kit, Manufacturer: Elabscience, Catalog No.: E-EL-0122C;
   2) Free Triiodothyronine (FT3) ELISA Kit, Manufacturer: Elabscience, Catalog No.: E-EL-0079C; 3) Rat Thyroid-Stimulating Hormone (TSH) ELISA Kit, Manufacturer: Elabscience, Catalog No.: E-EL-0079C.
(2) After 21 days of administration, the rats are sacrificed, and their thyroid tissues are collected for Hematoxylin and Eosin (HE) staining.

### 5. Experimental Results

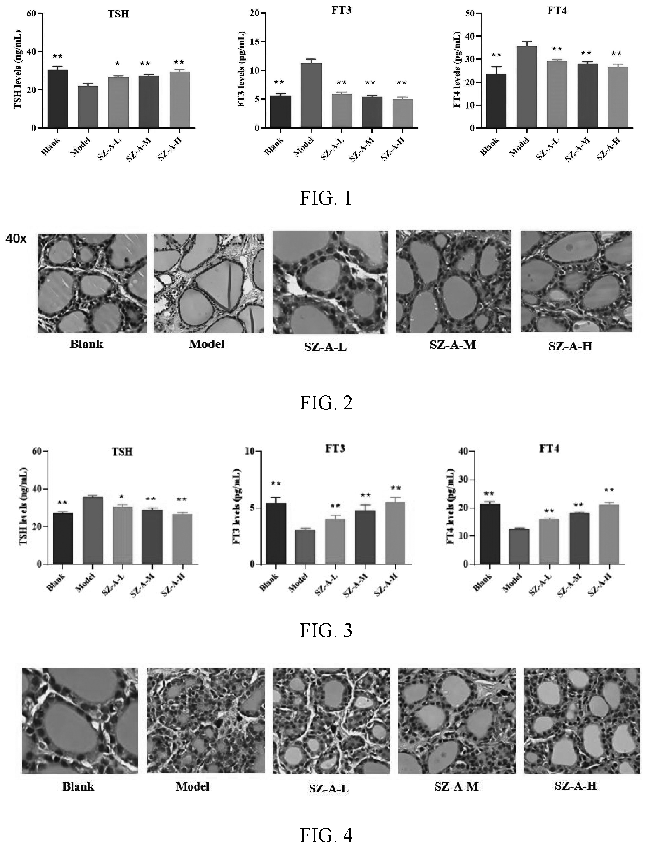

As can be seen from FIG. 1, compared with the Blank group, the Model group showed a significant decrease in serum thyroid-stimulating hormone (TSH) and a significant increase in free triiodothyronine (FT3) and free thyroxine (FT4), indicating hyperthyroidism in the Model group. After treatment with SZ-A, the low, medium, and high dose groups of SZ-A all significantly increased TSH expression and significantly decreased FT3 and FT4 expression. Furthermore, the improvement effect of SZ-A on TSH, FT3, and FT4 showed a certain dose-dependent relationship.

The results of HE staining of thyroid tissue are shown in FIG. 2. In the Blank group, the thyroid lobe tissue structure is clear, with follicles of varying sizes and shapes distributed throughout. The follicles are surrounded by a single layer of cuboidal epithelial cells, and the follicular lumens are filled with darkly stained, transparent colloid. In the Model group, the thyroid tissue structure is abnormal. The follicular epithelial cells are significantly thinner than those in the Blank group, the follicular lumens are enlarged, and there is obvious fusion of follicles.

In the SZ-A-L group, the size and structure of the thyroid follicles are significantly improved. The follicular epithelial cells are thicker than those in the model group, and fusion is rare. The thyroid tissues in the SZ-A-M and SZ-A-H dose groups are similar in structure to the blank group. The follicles are of varying sizes, surrounded by a single layer of cuboidal epithelial cells, and darkly stained, transparent colloid is visible in the lumens. This suggests that SZ-A has a good therapeutic effect on thyroid tissue lesions.

### Experimental Example 2

### 1. Experimental Drugs

DHEA (Dehydroepiandrosterone) oil solution: 30 mg/mL. 30 mg of DHEA is dissolved in 1 mL of castor oil and placed on a magnetic stirrer at 50°C with a rotation speed of 500 r/min until the DHEA is completely dissolved.

Mulberry extract SZ-A prepared in Preparation Example 1: This is formulated into different concentrations and dissolved in an aqueous solution.

### 2. The experimental animals are the same as in Experimental Example 1.

### 3. Modeling and Administration

The hypothalamic-pituitary-thyroid (HPT) axis and the hypothalamic-pituitary-ovarian (HPO) axis form a two-way loop. An abnormality in one axis can affect the normal function of the other. When the body is exposed to exogenous androgens in excess or given an inducer that inhibits androgen metabolism, leading to high androgen levels, the HPO axis is disrupted, which can cause hyperandrogenemia or Polycystic Ovary Syndrome (PCOS). This, in turn, affects the pituitary and thyroid functions regulated by the hypothalamus, leading to abnormal levels of thyroid-stimulating hormone (TSH) secreted by the pituitary or FT3 and FT4 secreted by the thyroid. Consequently, the body may develop various symptoms such as hyperthyroidism or hypothyroidism, as well as thyroid inflammation or nodules.

**Table 2**

| Group | Abbreviation | Number of Animals | Dosage (calculated as total mulberry branch alkaloids, mg/kg/day) | Route of Administration | Diet |
|---|---|---|---|---|---|
| Blank Group | Blank | 6 | 0 | | Chow |
| Model Group | Model | 6 | 0 | Oral gavage | HFD |
| Treatment Group | SZ-A-L | 6 | 67 | | |
| Treatment Group | SZ-A-M | 6 | 100 | | |
| Treatment Group | SZ-A-H | 6 | 150 | | |

Twenty-one-day-old, newly weaned, healthy female Sprague-Dawley (SD) rats with glossy coats are selected. After 3 days of adaptive feeding, they are randomly divided into a blank group, a model group, an SZ-A-L group, an SZ-A-M group, and an SZ-A-H group, with 6 rats in each group. The blank group is fed a standard chow diet and subcutaneously injected with the vehicle, castor oil (60 mg/kg/day). The model and treatment groups are fed a high-fat diet (HFD, Research Diets D12492) and simultaneously subcutaneously injected with DHEA oil solution (60 mg/kg/day) for 7 weeks to establish the rat model.

After the model is successfully evaluated (compared with the Blank group, the Model group showed a significant increase in serum thyroid-stimulating hormone (TSH) and a significant decrease in free triiodothyronine (FT3) and free thyroxine (FT4)), the blank group continued to receive the standard chow diet without any treatment. The model and treatment groups continued to receive the high-fat diet. The model group is orally gavaged with normal saline, the SZ-A-L group is orally gavaged with SZ-A (67 mg/kg/day), the SZ-A-M group is orally gavaged with SZ-A (100 mg/kg/day), and the SZ-A-H group is orally gavaged with SZ-A (150 mg/kg/day). After 21 days of continuous treatment, samples are collected for efficacy testing. The specific detection and analysis methods are the same as in Experimental Example 1.

### 4. Experimental Results

The results are shown in FIG. 3. Compared with the Blank group, the Model group showed a significant increase in serum thyroid-stimulating hormone (TSH) and a significant decrease in free triiodothyronine (FT3) and free thyroxine (FT4), indicating hypothyroidism in the Model group. After treatment with SZ-A, the low, medium, and high dose groups of SZ-A all significantly decreased TSH expression and significantly increased FT3 and FT4 expression. Furthermore, the improvement effect of SZ-A on TSH, FT3, and FT4 showed a certain dose-dependent relationship.

The results of HE staining of thyroid tissue are shown in FIG. 4. The HE staining results showed that in the Blank group, the thyroid tissue structure is clear, with follicles of varying sizes surrounded by a single layer of cuboidal epithelial cells, and the follicular lumens are filled with darkly stained, transparent colloid. In the Model group, the thyroid follicles showed diffuse nodular hyperplasia, mostly consisting of small cell follicles. The lumens contained little or no transparent colloid, and there is partial lymphocyte infiltration, suggesting hypothyroidism with mild inflammation.

In the SZ-A-L group, the nodular hyperplasia of thyroid follicles is reduced. Some are small cell follicles, and some are medium-sized follicles. The epithelial cells are a single layer of cuboidal cells, and secretion is observed in the lumens with no lymphocyte infiltration. This suggests that SZ-A-L had a certain improving effect on thyroid tissue lesions.

In the SZ-A-M group, the nodular hyperplasia of thyroid follicles is significantly reduced. The follicle size is similar to that of the Blank group, and light red transparent colloid is seen in the lumens with no lymphocyte infiltration.

In the SZ-A-H group, there are almost no follicular nodules. The gland showed irregularly shaped cell follicles of varying sizes, and a large amount of darkly stained transparent colloid is seen in the lumens. The structure is similar to that of the blank group, with no lymphocyte infiltration. This suggests that the improving effect of SZ-A on thyroid tissue has a certain dose-dependent relationship.

### Experimental Example 3

### 1. Experimental Drugs

DHEA (Dehydroepiandrosterone) oil solution: 30 mg/mL. 30 mg of DHEA is dissolved in 1 mL of castor oil and placed on a magnetic stirrer at 50°C with a rotation speed of 500 r/min until the DHEA is completely dissolved.

Mulberry extract SZ-A prepared in Preparation Example 1: This is formulated into different concentrations and dissolved in an aqueous solution.

### 2. Experimental Animals

Twenty-four 3-week-old, newly weaned, healthy female Sprague-Dawley (SD) rats with glossy coats are selected and housed under Specific Pathogen-Free (SPF) conditions.

### 3. Modeling and Administration

**Table 3**

| Group | Abbreviation | Number of Animals | Dosage (calculated as total mulberry branch alkaloids, mg/kg/day) | Route of Administration | Diet |
|---|---|---|---|---|---|
| Blank Group | Blank | 6 | 0 | | Chow |
| Model Group | Model | 6 | 0 | Oral gavage | HFD |
| Treatment Group | SZ-A-L | 6 | 67 | | |
| Treatment Group | SZ-A-H | 6 | 150 | | |

Twenty-one-day-old, newly weaned, healthy female Sprague-Dawley (SD) rats with glossy coats are selected. After 3 days of adaptive feeding, they are randomly divided into a blank group, a model group, an SZ-A-L group, and an SZ-A-H group, with 6 rats in each group. The blank group is fed a standard chow diet and subcutaneously injected with the vehicle, castor oil (60 mg/kg/day). The model and treatment groups are fed a high-fat diet (HFD, Research Diets D12492) and simultaneously subcutaneously injected with DHEA oil solution (60 mg/kg/day) for 7 weeks to establish the rat model.

After the model is successfully evaluated (compared with the Blank group, the Model group showed a significant increase in TPO-Ab and TG-Ab antibodies), the blank group continued to receive the standard chow diet without any treatment. The model and treatment groups continued to receive the high-fat diet. The model group is orally gavaged with normal saline, the SZ-A-L group is orally gavaged with SZ-A (67 mg/kg/day), and the SZ-A-H group is orally gavaged with SZ-A (150 mg/kg/day). After 21 days of continuous treatment, samples are collected for efficacy testing.

### 4. Experimental Results

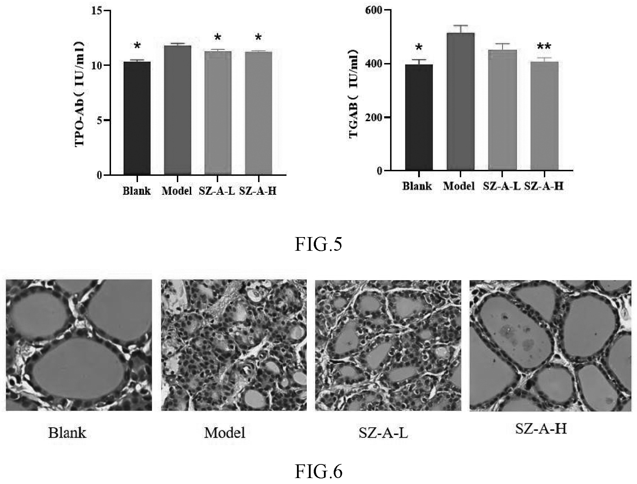

Serum is collected to detect the levels of TPO-Ab and TG-Ab antibodies using ELISA. The results are shown in FIG. 5. Compared with the Blank group, the Model group showed a significant increase in both TPO-Ab and TG-Ab, indicating an increase in thyroid-specific autoantibodies in the Model group. After treatment with SZ-A, both the low and high dose groups of SZ-A could reduce the expression of TPO-Ab and TG-Ab.

The results of HE staining of thyroid tissue are shown in FIG. 6. The HE staining results showed that in the Blank group, the thyroid tissue structure is clear, with follicles of varying sizes surrounded by a single layer of cuboidal epithelial cells, and the follicular lumens are filled with darkly stained, transparent colloid. In the Model group, the thyroid follicles showed severe diffuse nodular hyperplasia, mostly consisting of small cell follicles, with a large amount of lymphocyte infiltration, suggesting severe destruction of the thyroid tissue structure. In the SZ-A-L group, the hyperplasia of thyroid follicles is reduced, and some follicles recovered their normal structure with a single layer of cuboidal epithelial cells. Secretion is observed in the lumens, and there is no lymphocyte infiltration, suggesting that SZ-A-L had a certain improving effect on thyroid tissue lesions. In the SZ-A-H group, there is almost no follicular nodular hyperplasia. Follicles of varying sizes and irregular shapes are visible, and a large amount of darkly stained transparent colloid is seen in the lumens. The structure is similar to that of the blank group, with no lymphocyte infiltration, suggesting that SZ-A-H had a good improving effect on thyroid tissue.

The above research results suggest that SZ-A has a good therapeutic effect on Hashimoto's thyroiditis.

Experimental Example 4: Pharmacodynamic Test of Mulberry Extract in the Treatment of Hashimoto's Thyroiditis

### I. Animal Experiment Protocol

### 1.1 Experimental Animals

Lewis rats, 5-6 weeks old, weighing 120-140 g. After being housed in the animal facility, they are adaptively fed for 3 days. After weighing, they are randomly divided into 6 groups (designated as Day 1 of Week 1), with 10 rats in each group.

The experimental groups are: 1. Normal control group, 2. Model group, 3. Positive control group, 4-6. SZ-A groups with different dosages (Preparation Example 1, calculated based on alkaloids: low, medium, and high, with dosages of 67 mg/mL, 100 mg/mL, and 150 mg/mL).

### 1.2 Experimental Drugs

Sodium Iodide
Porcine Thyroglobulin
Freund's Complete Adjuvant
Freund's Incomplete Adjuvant
Levothyroxine Sodium
Mulberry extract from Preparation Example 8

### 1.3 Modeling and Administration

On the day of grouping, all groups except the normal control group are used to construct the HT model. On Day 1 of Week 1, 0.2 mL of porcine thyroglobulin (4 mg/mL) emulsified with Freund's complete adjuvant is injected into the rat footpads twice a week for initial immunization. In Week 2 and Week 4, a single booster immunization is performed by injecting an equal dose of porcine thyroglobulin emulsified with incomplete Freund's adjuvant. The success of modeling is determined based on changes in TPO-Ab and TG-Ab levels. During the modeling period, all rats except those in the normal control group drank 0.05% sodium iodide solution until the end of modeling.

After modeling is completed, drug administration began. Rats in Group 3 are orally gavaged with levothyroxine sodium. Rats in Groups 4-6 are orally gavaged with mulberry extract SZ-A. The remaining groups are orally gavaged with an equal volume of normal saline based on body weight. Administration is performed once daily for 4 weeks.

### 1.4 Sample Collection and Detection

24 hours after the final treatment, 3 mL of blood is collected from each rat, and serum is obtained by centrifugation for the detection of FT3, FT4, TSH, TPO-Ab, and TG-Ab levels. After blood collection, the rats are anesthetized and sacrificed, and their thyroid tissues are collected. The tissues from each group (10 rats per group) are divided into two parts. One part is fixed in 4% paraformaldehyde for HE staining, and the other part is stored in a -80°C freezer for Western blotting experiments.

TPO-Ab and TG-Ab levels in rat serum are detected by ELISA.

Pathological changes in rat thyroid tissue are examined by HE staining.

Experimental results indicated that: SZ-A could reduce diffuse hyperplasia of rat thyroid tissue and improve serum TPO-Ab and TG-Ab levels.

### II. Cell Experiment Protocol

### 2.1 Experimental Cells

Thyroid follicular epithelial cells (Nthy-ori3-1)

### 2.2 Experimental Drugs

Sodium Iodide
Interferon-γ (IFN-γ)
Fetal Bovine Serum (FBS)
RPMI 1640 Medium
Mulberry branch extract from Preparation Examples 1-8

### 2.3 Cell Culture and Modeling

### 2.3.1 Nthy-ori3-1 Cell Culture

The immortalized human thyroid follicular epithelial cell line (Nthy-ori3-1) is cultured in RPMI 1640 medium containing 10% FBS (with 100 IU/mL penicillin and 100 µg/mL streptomycin) in a constant temperature incubator at 37°C with 5% CO₂.

When Nthy-ori3-1 cells reached 85-90% confluence in the culture dish, the medium is discarded, and the cells are washed twice with 1×PBS. Then, 1 mL of 0.25% trypsin containing EDTA is added to the dish and placed in a 37°C incubator for digestion for about 1 minute. Under an inverted optical microscope, when the Nthy-ori3-1 cells began to round up, the gaps between cells gradually widened, and some cells started to float, 3 mL of medium is added to stop the trypsin digestion. The adherent Nthy-ori3-1 cells are gently pipetted up and down to detach them from the dish. The cell suspension is then transferred to a sterile 15 mL centrifuge tube and centrifuged at 1000 rpm for 5 min at room temperature. The supernatant is discarded, and the cells are resuspended in 4 mL of medium. 10 µL of the suspension is taken for cell counting using a hemocytometer under a microscope. The cells are seeded into 6-well plates at a density of 3×10⁵ cells/well with 2 mL of medium per well.

### 2.3.2 Thyroiditis Cell Modeling

After the thyroid follicular epithelial cells (Nthy-ori3-1) entered the logarithmic growth phase, the control group cells are cultured in normal medium, while the model group cells are cultured in medium containing IFN-γ. After 24 hours of continued culture, the cells are treated with mulberry branch extracts from different preparation examples (Preparation Examples 1-8) and cultured for another 24 hours.

Detection indicators: ROS detection, and inflammatory factor detection: TNF-α, IL-1β, IL-6.

Results: The mulberry branch extracts from Preparation Examples 1-8 could reduce the IFN-γ-induced increase in intracellular ROS and decrease the levels of TNF-α, IL-1β, and IL-6 in the cell supernatant to varying degrees.

### III. Human Experiment

The mulberry branch extract from Preparation Example 1 is taken, and an appropriate amount of excipients is added. The mixture is blended uniformly, and water is added to form a soft material. After granulation and drying, magnesium stearate is added, mixed uniformly, and compressed into tablets to obtain the mulberry branch extract preparation. Each tablet contained 50 mg of total alkaloids.

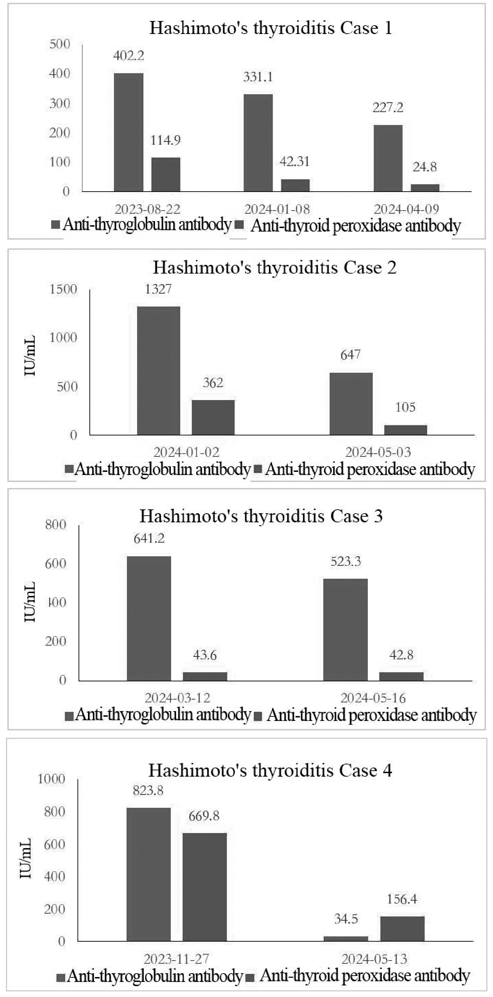

Clinical Cases and Treatment:
(1) Case 1: A 50-year-old Chinese female, weighing 65 kg, with a height of 160 cm, diagnosed with Hashimoto's thyroiditis for 6 years. She is orally administered tablets containing the mulberry extract from Preparation Example 1, two tablets at a time, three times a day. After 16 weeks of oral administration, her anti-thyroid peroxidase antibody (TPO-Ab) decreased to the normal range, and her anti-thyroglobulin antibody (TG-Ab) decreased by 17.7%. After 28 weeks of oral administration, TPO-Ab remained stable within the normal range, and TG-Ab decreased by 43.5% (see FIG. 7). The sensation of thyroid pain is significantly relieved.
(2) Case 2: A 50-year-old Chinese female, weighing 71 kg, with a height of 158 cm, diagnosed with Hashimoto's thyroiditis for 4 years. She is orally administered tablets containing the mulberry extract from Preparation Example 1, two tablets at a time, three times a day. After 16 weeks of oral administration, TPO-Ab and TG-Ab decreased by 71.0% and 51.2%, respectively (see FIG. 7).
(3) Case 3: A 35-year-old Chinese female, weighing 60 kg, with a height of 162 cm, diagnosed with Hashimoto's thyroiditis for 3 years. She is orally administered tablets containing the mulberry extract from Preparation Example 1, two tablets at a time, three times a day. After 8 weeks of oral administration, TG-Ab decreased by 18.4%, and TPO-Ab, which is originally normal, remained stable within the normal range after medication (see FIG. 7).
(4) Case 4: A 52-year-old Chinese female, weighing 62 kg, with a height of 163 cm, diagnosed with Hashimoto's thyroiditis for 7 years. She is orally administered tablets containing the mulberry extract from Preparation Example 1, two tablets at a time, three times a day. After 24 weeks of oral administration, TG-Ab decreased to the normal range, and TPO-Ab decreased by 76.6% (see FIG. 7).

The above research results suggest that the mulberry branch extract has a good therapeutic effect on Hashimoto's thyroiditis.

### IV. Cell Experiment Protocol

### 4.1 Experimental Cells

Thyroid follicular epithelial cells

### 4.2 Experimental Drugs

Interferon-γ (IFN-γ)
Fetal Bovine Serum (FBS)
RPMI 1640 Medium
Mulberry branch extracts from Preparation Examples 2-4, 6, 8, and 10.

### 4.3 Cell Culture and Modeling

### 4.3.1 Nthy-ori-3.1 Cell Culture

The immortalized human thyroid follicular epithelial cell line (Nthy-ori3-1) is cultured in RPMI 1640 medium containing 10% FBS (with 100 IU/mL penicillin and 100 µg/mL streptomycin) in a constant temperature incubator at 37°C with 5% CO₂.

When Nthy-ori3-1 cells reached 85-90% confluence in the culture dish, the medium is discarded, and the cells are washed twice with 1×PBS. Then, 1 mL of 0.25% trypsin containing EDTA is added to the dish and placed in a 37°C incubator for digestion for about 1 minute. Under an inverted optical microscope, when the Nthy-ori3-1 cells began to round up, the gaps between cells gradually widened, and some cells started to float, 3 mL of medium is added to stop the trypsin digestion. The adherent Nthy-ori3-1 cells are gently pipetted up and down to detach them from the dish. The cell suspension is then transferred to a sterile 15 mL centrifuge tube and centrifuged at 1000 rpm for 5 min at room temperature. The supernatant is discarded, and the cells are resuspended in 4 mL of medium. 10 µL of the suspension is taken for cell counting using a hemocytometer under a microscope. The cells are seeded into 48-well plates at a density of 1×10⁴ cells/well with 250 µL of medium per well.

### 4.3.2 Thyroiditis Cell Modeling

After the thyroid follicular epithelial cells (Nthy-ori3-1) entered the logarithmic growth phase, the control group cells are cultured in normal medium. The model group cells are cultured in medium containing 150 ng/mL IFN-γ. Simultaneously, the treatment groups are given mulberry branch extracts from different preparation examples (Preparation Examples 2-4, 6, 8, and 10) at a concentration of 150 µg/mL and cultured for 48 hours. The supernatant is collected and centrifuged at 4°C, 1000×g for 20 minutes to remove impurities and cell debris. The supernatant is then collected for testing.

According to the instructions for the ELISA kits for TNF-α, FT4, and IL-6, the centrifuged supernatant samples are processed. TNF-α and FT4 are detected in the original solution, while IL-6 is detected after a 40-fold dilution of the original solution. The absorbance (OD) of each well is measured at 450 nm using a microplate reader, and the contents of TNF-α, FT4, and IL-6 in the supernatant of each group are calculated based on the OD values. Human Tumor Necrosis Factor-α (TNF-α) ELISA Kit, Manufacturer: Elabscience, Catalog No.: E-EL-H0109. Free Thyroxine (fT4) ELISA Kit, Manufacturer: Elabscience, Catalog No.: E-EL-0122. Human Interleukin-6 (IL-6) ELISA Kit, Manufacturer: Elabscience, Catalog No.: E-EL-H6156.

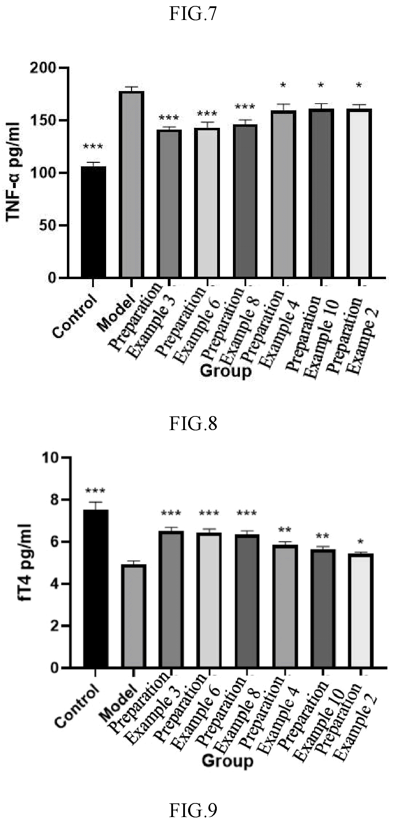

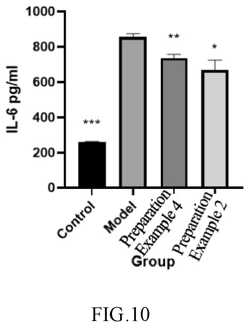

The results are shown in Figures 8, 9, and 10.

The results showed that the mulberry branch extracts from Preparation Examples 2-4, 6, 8, and 10 could reduce TNF-α in the cell supernatant and increase FT4 in the supernatant to varying degrees. The mulberry branch extracts from Preparation Examples 2 and 4 could reduce IL-6 in the cell supernatent to varying degrees. These cell-level experimental results prove that: (1) the mulberry branch extract can alleviate Hashimoto's thyroiditis; (2) the mulberry branch extract can alleviate hypothyroidism.

### Industrial Applicability

The present disclosure discloses the use of mulberry extract in the preparation of a medicament for improving and/or treating thyroid diseases. The present disclosure confirms through experiments that the mulberry extract can regulate the secretion levels of TSH, FT3, and FT4 in SD rats with hyperthyroidism or hypothyroidism, regulate the thyroid antibody levels in patients with Hashimoto's thyroiditis, and has a significant improving effect on thyroid tissue lesions. As a component derived from a natural plant, the mulberry extract of the present disclosure possesses the unique advantages of low toxicity and side effects, as well as mild and long-lasting effects. Therefore, the mulberry extract, with its significantly enhanced medication safety, has great advantages in the treatment of thyroid diseases.

## Claims

1. Use of mulberry extract or a main active ingredient thereof, selected from any of the following (a1) to (a4):
(a1) use of mulberry extract in preparing a product for preventing and/or treating thyroid disease;
(a2) use of mulberry extract in preparing a product for improving thyroid disease;
(a3) use of mulberry extract in preventing and/or treating thyroid disease;
(a4) use of mulberry extract in improving thyroid disease.

2. The use according to claim 1, wherein the thyroid disease comprises at least one of the following: hypothyroidism, hyperthyroidism, thyroid nodule, and thyroiditis;
preferably, the thyroiditis is Hashimoto's thyroiditis.

3. The use according to claim 1 or 2, wherein treating or improving of thyroid disease comprises:
1) regulating a secretion level of serum thyroid-stimulating hormone in a patient with thyroid disease;
2) regulating a secretion level of free triiodothyronine in a patient with thyroid disease;
3) regulatinga secretion level of free thyroxine in a patient with thyroid disease;
4) improving thyroid tissue lesions in a patient with thyroid disease;
5) improving a level of thyroid antibodies in a patient with thyroid disease;
6) reducing a level of reactive oxygen species (ROS) in a patient with thyroid disease;
7) improving a level of inflammatory factors in a patient with thyroid disease.

4. The use according to claim 1 or 2, wherein
the thyroid disease is hypothyroidism;
or the hypothyroidism comprises: an increase in the level of serum thyroid-stimulating hormone;
or the hypothyroidism comprises: an increase in the level of serum thyroid-stimulating hormone and a decrease in the level of free thyroxine;
or the hypothyroidism comprises: an increase in the level of serum thyroid-stimulating hormone and decreases in levels of free triiodothyronine and free thyroxine;
or the preventing and/or treating and/or improving of hypothyroidism comprises least one of the following: reducing the level of serum thyroid-stimulating hormone, increasing levels of free triiodothyronine, and increasing levels of free thyroxine;
or the hypothyroidism comprises a disorder of the serum thyroid-stimulating hormone level, with or without a decrease in the level of free triiodothyronine and/or free thyroxine; the disorder of the serum thyroid-stimulating hormone level comprises an increase or decrease in the level of serum thyroid-stimulating hormone;
or the hypothyroidism comprises a normal level of serum thyroid-stimulating hormone and a decrease in the level of free thyroxine, with or without a decrease in the level of free triiodothyronine;
or the hypothyroidism comprises a decrease in the level of serum thyroid-stimulating hormone and decreases in the levels of free triiodothyronine and free thyroxine;
or the hypothyroidism comprises a normal level of serum thyroid-stimulating hormone and a decrease in the level of free thyroxine;
or the hypothyroidism comprises a normal level of serum thyroid-stimulating hormone and decreases in the levels of free triiodothyronine and free thyroxine;
or the preventing and/or treating and/or improving of hypothyroidism comprises regulating the level of serum thyroid-stimulating hormone, and/or increasing the level of free triiodothyronine, and/or increasing the level of free thyroxine; the regulating is increasing, decreasing, or maintaining a normal level.

5. The use according to claim 1 or 2, wherein
the thyroid disease is hyperthyroidism;
or the hyperthyroidism comprises a decrease in the level of serum thyroid-stimulating hormone;
or the hyperthyroidism comprises a decrease in the level of serum thyroid-stimulating hormone and an increase in the level of free thyroxine;
or the hyperthyroidism comprises a decrease in the level of serum thyroid-stimulating hormone and increases in the levels of free triiodothyronine and free thyroxine;
or the preventing and/or treating and/or improving of hyperthyroidism comprises at least one of the following: increasing the level of serum thyroid-stimulating hormone and decreasing the levels of triiodothyronine and free thyroxine;
or the hyperthyroidism comprises a disorder of the serum thyroid-stimulating hormone level, with or without an increase in the level of free triiodothyronine and/or free thyroxine; the disorder of the TSH level comprises an increase or decrease in the TSH level;
or the hyperthyroidism comprises a normal level of serum thyroid-stimulating hormone and increases in the levels of free triiodothyronine and free thyroxine;
or the hyperthyroidism comprises a decrease in the level of serum thyroid-stimulating hormone and an increase in the level of free triiodothyronine;
or the hyperthyroidism comprises an increase in the level of serum thyroid-stimulating hormone and increases in the levels of free triiodothyronine and free thyroxine;
or the hyperthyroidism comprises a normal level of serum thyroid-stimulating hormone and increases in the levels of free triiodothyronine and free thyroxine;
or the preventing and/or treating and/or improving of hyperthyroidism comprises regulating the level of serum thyroid-stimulating hormone, and/or decreasing the level of free triiodothyronine, and/or decreasing the level of free thyroxine; the regulating is increasing, decreasing, or maintaining a normal level.

6. The use according to claim 1 or 2, wherein
the thyroid disease comprises Hashimoto's thyroiditis;
or the Hashimoto's thyroiditis comprises at least one of the following: a significant increase in the levels of serum TPO-Ab and TG-Ab antibodies;
or the preventing and/or treating and/or improving of Hashimoto's thyroiditis comprises at least one of the following: reducing the level of serum TPO-Ab and reducing the level of serum TG-Ab antibodies.

7. Use of mulberry extract or a main active ingredient thereof, which is at least one of the following (b1) to (b7):
(b1) preparing a product for regulating a secretion level of serum thyroid-stimulating hormone in a patient with thyroid disease;
(b2) preparing a product for regulating a secretion level of free triiodothyronine in a patient with thyroid disease;
(b3) preparing a product for regulating a secretion level of free thyroxine in a patient with thyroid disease;
(b4) preparing a product for improving thyroid tissue lesions in a patient with thyroid disease;
(b5) preparing a product for improving a level of thyroid antibodies in a patient with thyroid disease;
(b6) preparing a product for reducing the level of reactive oxygen species (ROS) in a patient with thyroid disease;
(b7) preparing a product for improving a level of inflammatory factors in a patient with thyroid disease.

8. The use according to any one of claims 1 to 7, wherein the mulberry extract is mulberry branch extract, mulberry root bark extract, and/or mulberry leaf extract;
the preparation method of the mulberry extract comprises the following steps:
1) preparing a crude extract of Moraceae plants;
2) separating the crude extract with cation resin and/or optional anion resin to obtain the mulberry extract;
the method further comprises the following steps:
3) performing alcohol precipitation on the resin effluent from step 2) and collecting the supernatant;
4) performing concentration and drying on the supernatant;
or the method further comprises the following step: performing concentration and drying on the resin effluent from step 2).

9. The use according to any one of claims 1 to 8, wherein the main active ingredient of the mulberry extract comprises at least one of the following: 1-deoxynojirimycin, N-methyl-1-deoxynojirimycin, fagomine, 3-epifagomine, 1,4-dideoxy-1,4-imino-D-ribitol, calystegine B2, calystegine C1, 2-O-(α-D-galactopyranosyl)-1-deoxynojirimycin, 6-O-(β-D-glucopyranosyl)-1-deoxynojirimycin, and 1,4-dideoxy-1,4-imino-(2-O-β-D-glucopyranosyl)-D-arabitol;
or the mulberry extract acts on humans or mammals.

10. A method for preventing and/or treating and/or improving thyroid disease, comprising the following step: administering the mulberry extract or the main active ingredient thereof according to any one of claims 1 to 9 to a recipient animal or human to prevent and/or treat and/or improve thyroid disease.
